(19) **Europäisches Patentamt / European Patent Office / Office européen des brevets**

(11) **EP 3 987 229 B1**

(12) **EUROPÄISCHE PATENTSCHRIFT**

(45) Veröffentlichungstag und Bekanntmachung des Hinweises auf die Patenterteilung:
**18.02.2026 Patentblatt 2026/08**

(21) Anmeldenummer: **20739238.2**

(22) Anmeldetag: **17.06.2020**

(51) Internationale Patentklassifikation (IPC):
**F23G 5/50** *(2006.01)*

(52) Gemeinsame Patentklassifikation (CPC):
**F23G 5/50;** F23G 2207/20; F23G 2900/55003;
F23N 2900/05001

(86) Internationale Anmeldenummer:
**PCT/AT2020/060246**

(87) Internationale Veröffentlichungsnummer:
**WO 2020/252513 (24.12.2020 Gazette 2020/52)**

(54) **VERFAHREN ZUR ANALYSE DES BETRIEBS UND ZUR BETRIEBSOPTIMIERUNG VON MÜLLVERBRENNUNGSANLAGEN**

METHOD FOR ANALYZING AND OPTIMIZING THE OPERATION OF WASTE INCINERATOR SYSTEMS

PROCÉDÉ D'ANALYSE DU FONCTIONNEMENT ET D'OPTIMISATION DU FONCTIONNEMENT D'INSTALLATIONS D'INCINÉRATION D'ORDURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.06.2019 AT 505552019**

(43) Veröffentlichungstag der Anmeldung:
**27.04.2022 Patentblatt 2022/17**

(73) Patentinhaber: **TECHNISCHE UNIVERSITÄT WIEN**
**1040 Wien (AT)**

(72) Erfinder:
• **FELLNER, Johann**
**1140 Wien (AT)**
• **SCHWARZBÖCK, Therese**
**1200 Wien (AT)**

(74) Vertreter: **Patentanwälte**
**Barger, Piso & Partner**
**Operngasse 4**
**1010 Wien (AT)**

(56) Entgegenhaltungen:
EP-A1- 0 317 731    EP-A2- 1 698 827
EP-A2- 1 715 339

**EP 3 987 229 B1**

## Beschreibung

Die Erfindung betrifft ein Verfahren zur Analyse des Betriebs und zur Betriebsoptimierung von Müllverbrennungsanlagen entsprechend den Oberbegriffen der Ansprüche 1 und 2.

Aus der EP 1 698 827 ist es bekannt, den $CO_2$ Gehalt des Abgases z.B. einer Müllverbrennungsanlage zu bestimmen, um daraus auf die "Verbrennungsintensität" zu schließen, und bei Überschreiten eines gewissen Levels das Verhältnis der Sauerstoffmassenströme von Primärverbrennungsgas und Sekundärverbrennungsgas so zu regeln, dass sie wieder unter dieses Level sinkt.

Aus der EP 1 715 339 der Anmelderin ist es bekannt, zur Ermittlung der Anteile biogener und fossiler Energieträger einer Müllverbrennungsanlage zwingend die Abgleichung zumindest dreier Bilanzen, ausgewählt aus acht Bilanzen, vorzunehmen. Dieses Verfahren ist sehr genau, benötigt aber einen gewissen apparativen und Verfahrensaufwand.

In Müllverbrennungsanlagen wird, wie der Name schon sagt, Müll verbrannt und damit ein Brennstoff, der sehr heterogen zusammengesetzt ist und insbesondere ein schwankendes Verhältnis von biogenen Abfallbestandteilen zu fossilen Abfallbestandteilen, zu denen auch Kunststoffe aller Art zählen, aufweist. Jede Müllverbrennungsanlage ist auf ein durch Untersuchungen zu erwartendes Verhältnis dieser beiden Anteile ausgelegt und weist beim Vorliegen dieses Verhältnisses die besten Betriebsergebnisse auf. Durch die völlig erratische Zufuhr des Abfalls, dies gilt auch, wenn bei der Beschickung des Bunkers und bei der Umwälzung des Abfalls im Bunker, aus dem die Brennkammer der Müllverbrennungsanlage ihr Brennmaterial erhält, aufgrund von Erfahrungswerten eine möglichst gleichmäßige Durchmischung angestrebt wird, kommt es zu unerwünschten zeitlichen Änderungen der Brennstoffzusammensetzung in der Brennkammer, die sich auf den Verbrennungsprozess auswirkt, auf die gewonnene (umgewandelte) Energie und auch auf die Abgaseigenschaften, wo diese Änderungen am besten manifest gemacht werden können.

Dazu ist noch festzuhalten: Ein genau definiertes Verhältnis von biogen zu fossilen Kohlenstoff als Auslegungsparameter für Müllverbrennungsanlagen gibt es nicht wirklich. Es ist auch betriebstechnisch kein Problem, die Anlage mit einem konstanten anderen Verhältnis von biogen zu fossil zu betreiben. Was problematisch ist, sind kurzfristige Änderungen in der Zusammensetzung des Brennstoffs.

So ist bei nahezu allen Arten fossiler Brennstoffe, bezogen auf stöchiometrische Luftzufuhr (somit 0 Vol.-% Sauerstoff im Abgas) der erhaltene $CO_2$-Anteil zwischen 15 und 17,6 Vol.-% wenn Erdgas und Methan, die ja in einer Müllverbrennung (mit Ausnahme von Stützbrennstoffen) kaum vorkommen, außer Acht gelassen werden. Im Vergleich dazu erhält man bei typischen biogenen Brennstoffen einen $CO_2$ Anteil im Abgas (bei stöchiometrische Luftzufuhr) zwischen 19,1 (Küchenabfälle) und 21 Vol.-% (Zellulose), wie aus der unten stehenden Tabelle 1 ersichtlich ist.

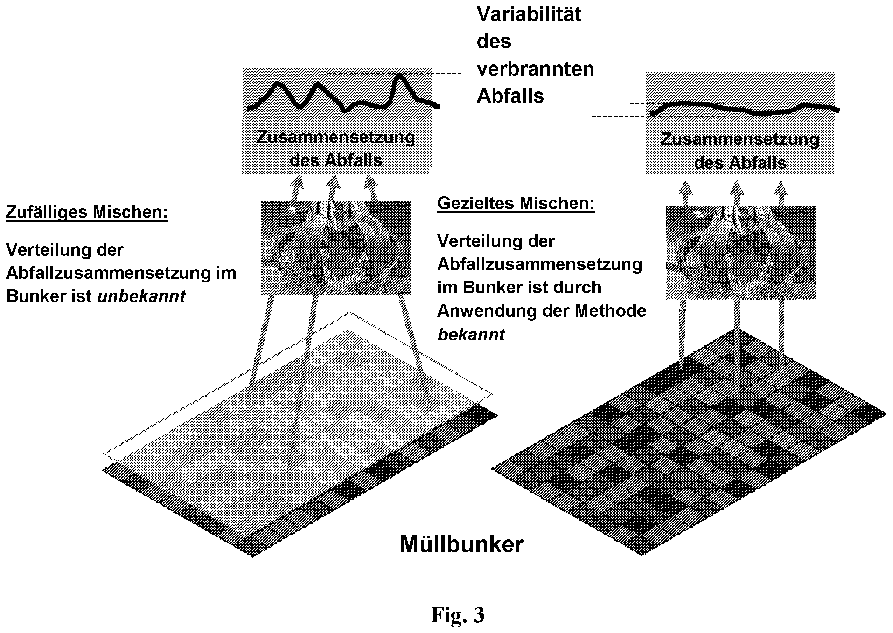

Unterschiede in der Abfallzusammensetzung und die sich durch die zufällige Zufuhr ergebenden zeitlichen Schwankungen sind deshalb unangenehm, weil Müllverbrennungsanlagen üblicherweise auch zur Energiegewinnung (eigentlich Umwandlung) eingesetzt werden, und dazu eine möglichst gleichmäßige Menge an Dampf (mit gleichbleibender Temperatur und gleichbleibendem Druck) pro Stunde erzeugt werden soll. Dies erreicht man im Stand der Technik teilweise über eine automatisierte oder manuell gesteuerte Durchmischung des angelieferten Abfalls im Müllbunker mithilfe des Bunkerkrans (siehe Fig. 3 - linke Abbildung), im Extremfall durch Zufuhr von Heizöl oder Erdgas zur Aufrechterhaltung einer ausreichenden Energieumwandlung oder durch in Kauf nehmen von Verlusten durch eine Teilauslastung der Anlage.

Aus vorhandenen Messungen und Untersuchungen an verschiedenen Anlagen geht hervor, dass bei einer Müllverbrennungsanlage mit einer Jahreskapazität von 200.000 Tonnen durch insbesondere kurzzeitige Schwankungen der Zusammensetzung des zugeführten Brennstoffs zwischen € 200.000,-- und € 500.000,-- nicht lukriert werden können bzw. wegen der Zusatzbrennstoffe (üblicherweise Erdgas oder Heizöl) aufgewandt werden müssen, was nicht nur pro Anlage einen hohen Betrag darstellt, sondern durch die Tatsache, dass es in Europa etwa 400 Müllverbrennungsanlagen einer solchen Größe gibt, zu einer beträchtlichen wirtschaftlichen Größe wird.

Es ist Ziel und Aufgabe der Erfindung, ein Verfahren der eingangs genannten Art anzugeben, mit dem die Variationen im Betrieb verringert werden können.

Erfindungsgemäß geschieht dies alternativ oder kumulativ durch:

- Ein Verfahren zur Analyse des Betriebs von Müllverbrennungsanlagen, wobei im Abgas der Gehalt an $CO_2$ gemessen und nach Rückführung auf die Größe $CO_{2Bezug}$ das Verhältnis von biogenem zu fossilen Kohlenstoff im verbrannten Müll daraus bestimmt wird und die Variabilität von $CO_{2Bezug}$ bzw. des Verhältnisses von biogenem zu fossilen Kohlenstoff im verbrannten Müll nach Größe und Dauer bestimmt und aufgezeichnet werden.
- Ein Verfahren zur Betriebsoptimierung von Müllverbrennungsanlagen, wobei im Abgas der Gehalt an $CO_2$ gemessen und nach Rückführung auf die Größe $CO_{2Bezug}$ das Verhältnis von biogenem zu fossilen Kohlenstoff im verbrannten Müll daraus bestimmt wird und die Variabilität von $CO_{2Bezug}$ bzw. des Verhältnisses von biogenem zu fossilen Kohlenstoff im verbrannten Müll nach Größe und Dauer bestimmt und zur Auswahl des der Verbrennung zuzuführenden Mülls herangezogen wird.

- Ein Verfahren zur Betriebsoptimierung von Müllverbrennungsanlagen, wobei im Abgas der Gehalt an $CO_2$ gemessen und nach Rückführung auf die Größe $CO_{2Bezug}$ das Verhältnis von biogenem zu fossilen Kohlenstoff im verbrannten Müll daraus bestimmt wird und die Variabilität von $CO_{2Bezug}$ bzw. des Verhältnisses von biogenem zu fossilen Kohlenstoff im verbrannten Müll nach Größe und Dauer bestimmt und zur Durchmischung des im Annahmebunker lagernden Mülls verwendet wird.

Die Erfindung wird im Folgenden anhand der Zeichnung näher beschrieben. Dabei zeigt bzw. zeigen

die Fig. 1 den Zusammenhang zwischen der Abgaszusammensetzung und der Brennstoffzusammensetzung im Hinblick auf biogenes bzw. fossiles Material,
die Fig. 2 ein Beispiel für eine Echtzeitanalyse der Zusammensetzung des Abfallinputs,
die Fig. 3 in zwei schematischen Darstellungen die Auswirkung des erfindungsgemäß optimierten Mischens,
die Fig. 4a und Fig. 4b eine Analyse der Variabilität der Brennstoffzusammensetzung zweier Müllverbrennungsanlagen,
die Fig. 5a und Fig. 5b den mittleren Verbrauch an Zusatzbrennstoff (am Beispiel Heizöl) und die mittlere Dampfproduktion einer Müllverbrennungsanlage, jeweils in Abhängigkeit von der Variabilität der Brennstoffzusammensetzung
die Fig. 6a und Fig. 6b den mittleren Abfalldurchsatz und die mittlere $O_2$-Konzentration im Abgas einer Müllverbrennungsanlage, jeweils in Abhängigkeit von der Variabilität der Brennstoffzusammensetzung.

Bevor auf die einzelnen Darstellungen in der Zeichnung näher eingegangen wird, sollen die theoretischen Grundlagen der Erfindung dargelegt werden:
Die Verbrennung unterschiedlicher Brennstoffe ist mit einer jeweils charakteristischen Abgaszusammensetzung (Gehalt an $O_2$ und $CO_2$ im trockenen Abgas, der kann vom gemessenen Gehalt im feuchten Abgas rückgerechnet werden, ist aber im Gegensatz dazu direkt vergleichbar und aussagekräftig) verbunden, wobei diese neben der chemischen Zusammensetzung des Brennstoffs (Gehalt an Wasser, C, H, O, N, S, F, Cl, ...) auch von der Menge der Verbrennungsluft abhängig ist. Beispielsweise führt eine höhere Menge an Verbrennungsluft bei einem bestimmten Brennstoff zu einer höheren $O_2$ Konzentration und einer geringeren $CO_2$ Konzentration im Abgas.
Durch eine rechnerische Normierung der Abgaszusammensetzung auf einen konstanten Sauerstoffgehalt im Abgas (z.B. Restsauerstoffgehalt von 0 % bei stöchiometrischem Luftbedarf oder konstanter Luftverhältniszahl) sind Änderungen in der Abgaszusammensetzung ausschließlich vom Brennstoff bzw. dessen chemischer Zusammensetzung abhängig.
Diese Normierung der Abgaszusammensetzung (auf einen beliebigen Sauerstoffgehalt im Abgas $O_{2Bezug}$) erfolgt durch folgende allgemein bekannte Gleichung, die es erlaubt, die trockene Abgaszusammensetzung für einen konstanten Sauerstoffgehalt im Abgas näherungsweise zu berechnen.

$$CO_{2Bezug} = \frac{CO_{2gemessen} \cdot \left(21 - O_{2Bezug}\right)}{\left(21 - O_{2gemessen}\right)}$$

$CO_{2Bezug}$     $CO_2$ Konzentration im trockenen Abgas bei konstanter (beliebig festzulegender) Luftverhältniszahl bzw. bei $O_{2Bezug}$ [Vol.-%]

$CO_{2gemessen}$     gemessene $CO_2$ Konzentration im trockenen Abgas der Verbrennungsanlage [Vol.-%]

$O_{2gemessen}$     gemessene $O_2$ Konzentration im trockenen Abgas der Verbrennungsanlage [Vol.-%]

$O_{2Bezug}$     beliebig festzulegender (konstanter) Sauerstoffgehalt im trockenen Abgas der Verbrennungsanlage (vorzugsweise wird ein Wert von 0 Vol.-% gewählt) [Vol.-%]

21     steht für $O_{2atm}$, den Sauerstoffgehalt der Atmosphäre [Vol.-%]

Für eine exakte Berechnung (unter Berücksichtigung des vorhandenen $CO_2$ Gehalts in der Verbrennungsluft bzw. in der Atmosphäre) der normierten $CO_2$ Konzentration $CO_{2Bezug}$ im trockenen Abgas bei konstanter (beliebig festzulegender) Luftverhältniszahl bzw. bei $O_{2Bezug}$ ist folgende Formel zu verwenden, wobei signifikante Unterschiede zwischen den Ergebnissen der Näherungsformel und der exakten Formel erst bei höheren Luftverhältniszahlen auftreten.

$$CO_{2_{Bezug}} = \left( CO_{2_{gemessen}} - \frac{CO_{2_{atm}} \cdot \left( 100\% - CO_{2_{gemesen}} - O_{2_{gemessen}} \right)}{\left( 100\% - O_{2_{atm}} - CO_{2_{atm}} \right)} \right) \cdot \frac{\left( O_{2_{atm}} - O_{2_{Bezug}} \right)}{\left( O_{2_{atm}} - O_{2_{gemessen}} \right)}$$

$$+ CO_{2_{atm}} \cdot$$

$$\cdot \left( 100\% - \left( CO_{2_{gemessen}} - \frac{CO_{2_{atm}} \cdot \left( 100\% - CO_{2_{gemesen}} - O_{2_{gemessen}} \right)}{\left( 100\% - O_{2_{atm}} - CO_{2_{atm}} \right)} \right) \cdot \right.$$

$$\left. \cdot \frac{\left( O_{2_{atm}} - O_{2_{Bezug}} \right)}{\left( O_{2_{atm}} - O_{2_{gemessen}} \right)} \right)$$

$O_{2_{atm}}$  $O_2$ Konzentration in der Verbrennungsluft bzw. in der Atmosphäre [Vol.-%], typischerweise liegt diese bei rund 20,94 Vol-%

$CO_{2_{atm}}$  $CO_2$ Konzentration in der Verbrennungsluft bzw. in der Atmosphäre [Vol.-%], typischerweise liegt diese bei rund 0,04 Vol-%

Die auf einen konstanten Sauerstoffgehalt im Abgas normierte trockene Abgaszusammensetzung $CO_{2_{Bezug}}$ und $O_{2_{Bezug}}$ ist, wie oben bereits erwähnt, ausschließlich vom Brennstoff bzw. dessen chemischer Zusammensetzung abhängig.

Zeitliche Variationen in der auf einen konstanten Sauerstoffgehalt $O_{2_{Bezug}}$ bezogenen (normierten) trockenen Abgaszusammensetzung (Gehalt an $CO_{2_{Bezug}}$) sind demzufolge das Resultat eines (zeitlich) in der Zusammensetzung geänderten Brennstoffs.

Demzufolge lässt sich bei Müllverbrennungsanlagen aus der zeitlichen Variation von $CO_{2_{Bezug}}$ auf die Homogenität bzw. Durchmischung des Müllinputs schließen. Ein nahezu konstanter Wert von $CO_{2_{Bezug}}$ deutet auf eine gute Durchmischung und konstante Zusammensetzung des Abfallinputs hin, während (kurzzeitige) Änderungen von $CO_{2_{Bezug}}$ auf eine schwankende Abfallzusammensetzung (und damit unzureichende Durchmischung) hinweisen.

Aus der zeitlichen Variation von $CO_{2_{Bezug}}$ ist eine Kontrolle und damit auch Steuerung der Bunkermülldurchmischung möglich.

Ziel des Müllverbrennungsanlagenbetreibers ist es, eine möglichst konstante (geringe Schwankungen) Zusammensetzung des Abfallinputs sicherzustellen, da nur dadurch ein optimaler (energieeffizienter) Betrieb gewährleistet werden kann.

Die schon eingangs angesprochene, nachfolgende Tabelle 1 zeigt Beispiele für normierte (auf 0 Vol.-% Sauerstoff bezogene) Abgaszusammensetzung unterschiedlicher Brennstoffe, ausgedrückt durch $CO_{2_{Bezug}}$.

Tabelle 1:

| Brennstoff bzw. Abfall | $O_{2_{Bezug}}$ [Vol.-%] | $CO_{2_{Bezug}}$ [Vol.-%] |
|---|---|---|
| Fossile Brennstoffe bzw. Abfälle | | |
| Erdgas | 0 | 12 |
| Methan | 0 | 11,8 |
| Polyethlyen | 0 | 15,1 |
| Polypropylen | 0 | 15,1 |
| PVC | 0 | 17,2 |
| Polystyrol | 0 | 17,6 |
| Heizöl EL | 0 | 15,6 |
| Biogene Brennstoffe bzw. Abfälle | | |
| Zellulose | 0 | 21 |
| Holz | 0 | 20,5 |
| Papier & Karton | 0 | 20,1 |
| Gartenabfälle | 0 | 19,6 |

(fortgesetzt)

| Biogene Brennstoffe bzw. Abfälle | | |
|---|---|---|
| *Küchenabfälle* | 0 | 19,1 |

Aus obiger Tabelle 1 ist ersichtlich, dass biogene Brennstoffe/Abfälle im Vergleich zu fossilen Brennstoffen einen höheren Wert von $CO_{2Bezug}$ aufweisen. D.h. vereinfacht kann die normierte Abgaszusammensetzung (ausgedrückt durch $CO_{2Bezug}$) auch verwendet werden um auf den Anteil an biogenen bzw. fossilen Materialien im Input von Müllverbrennungsanlagen zu schließen.

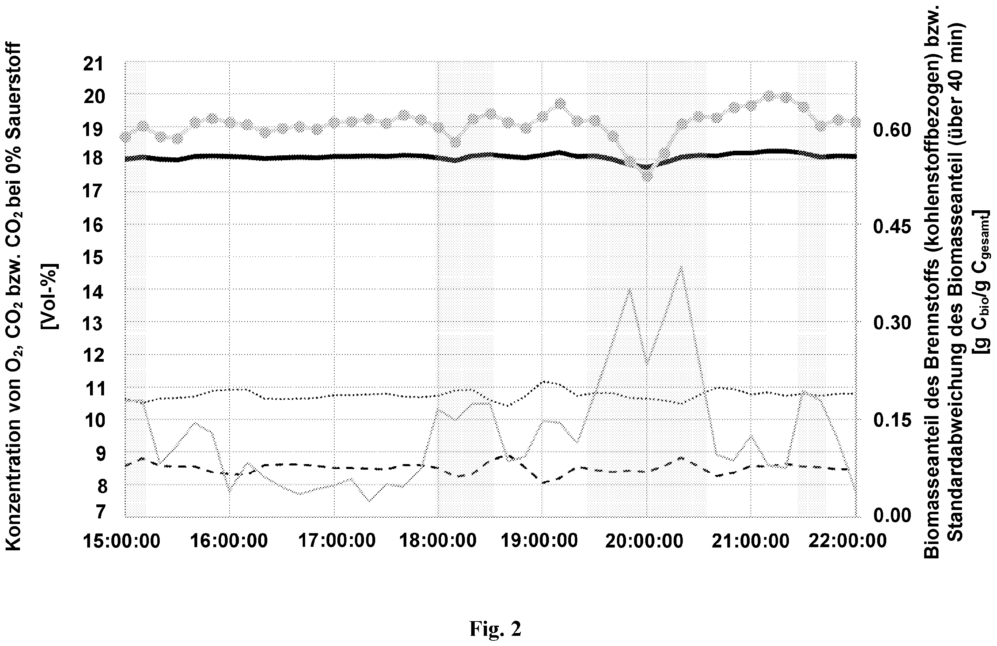

Nutzung der Erfindung zur Bewertung des Anlagenbetriebs unter Berücksichtigung der Mülldurchmischung: Mithilfe des erfindungsgemäßen Verfahrens lässt sich nicht nur die aktuelle Durchmischung/Homogenisierung des Bunkermülls kontrollieren und darauf basierend steuern (siehe Figs. 2 und 3), sondern das Verfahren eignet sich auch dazu, den Anlagenbetrieb (mit Bezug auf die Durchmischung/Homogenisierung des Bunkermülls) rückwirkend zu analysieren und den Einfluss einer unzureichenden Durchmischung/Homogenisierung des Abfalls auf den Betrieb zu quantifizieren.

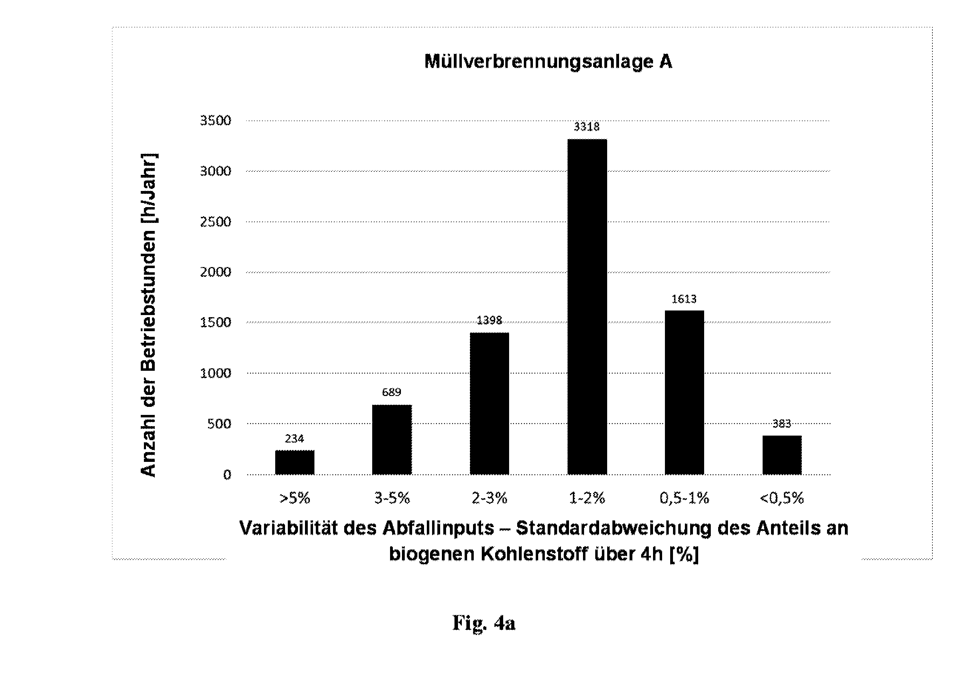

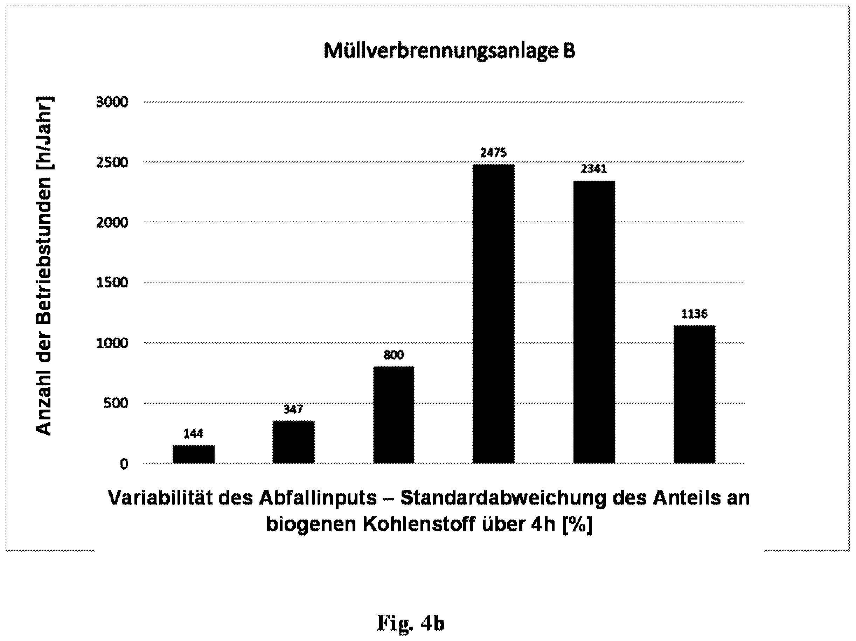

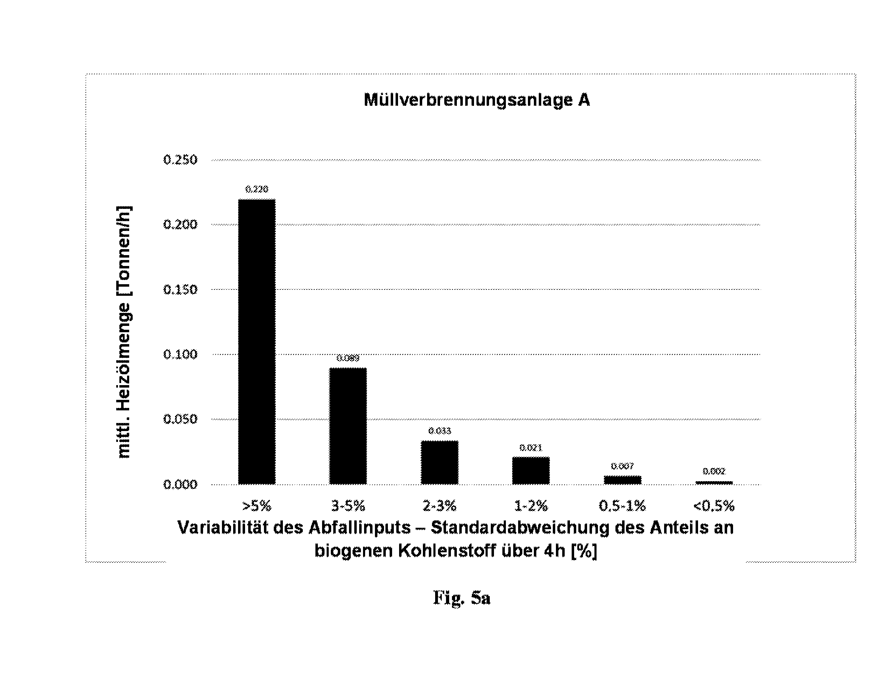

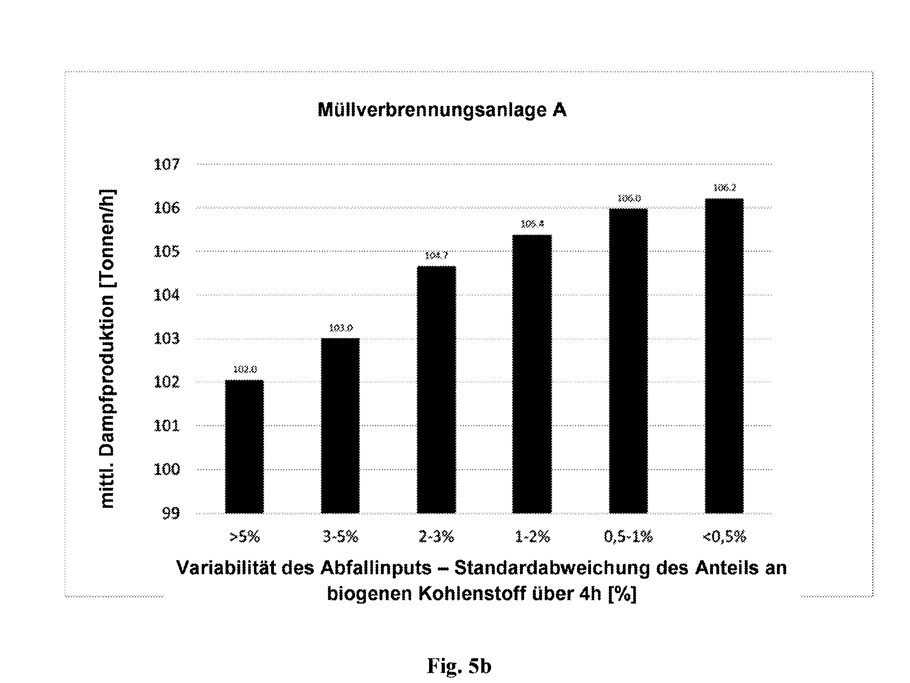

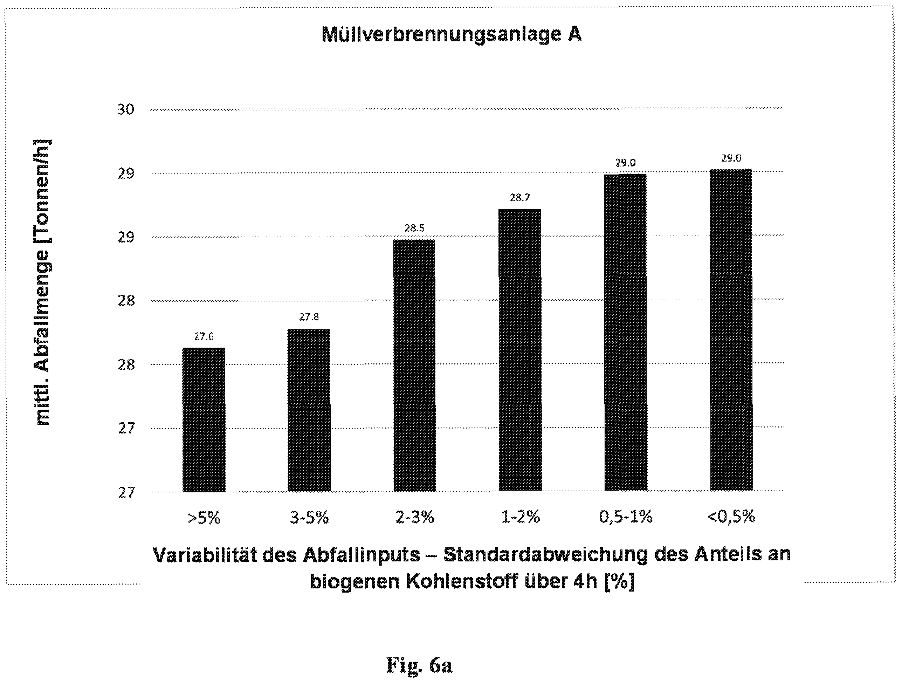

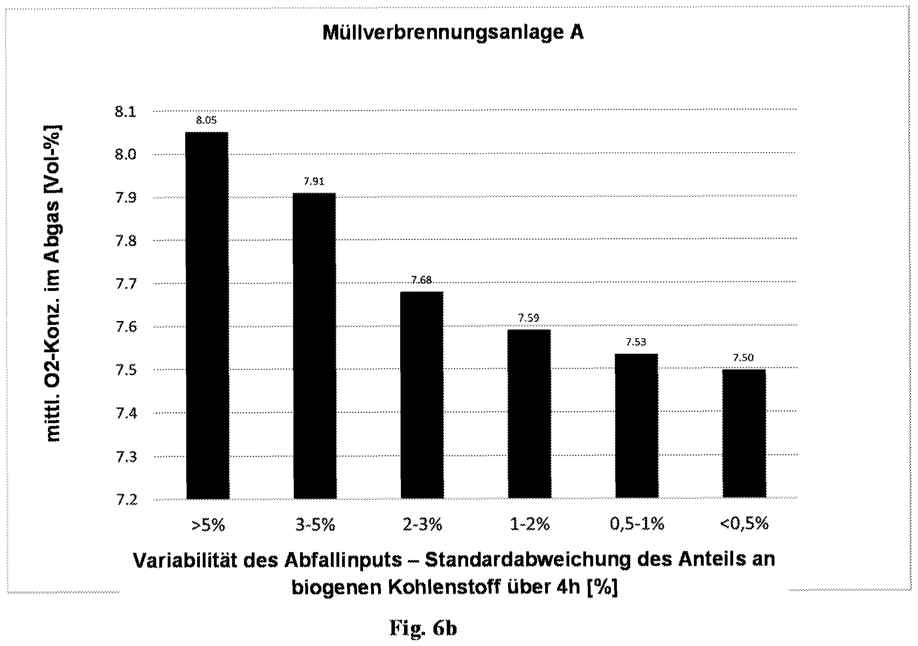

Beispielsweise lässt sich der Anteil an Betriebsstunden mit sehr guter bzw. schlechter Durchmischung/Homogenisierung des Bunkermülls eruieren (siehe Fig. 4). Durch die statistische Auswertung (z.B. Mittelwertbildung) von betriebsrelevanten Parametern der Müllverbrennungsanlage (z.B. Dampfproduktion, Abfalldurchsatz, Sauerstoffgehalt im Abgas, Verbrauch an Zusatzbrennstoffen) für die jeweils unterschiedlichen Zeiträume (Betriebsstunden mit sehr guter, guter, ... schlechter Durchmischung/Homogenisierung des Bunkermülls) lassen sich dann Aussagen über den Einfluss der Bunkermülldurchmischung auf den Betrieb machen (siehe Figs. 5 und 6). Der Anlagenbetreiber erhält dadurch wertvolle Informationen über das mögliche Optimierungspotential seiner Anlage durch eine bessere Durchmischung/-Homogenisierung des Bunkermülls.

Aus den Analysen für 2 Müllverbrennungsanlagen (Anlage A und B) zeigt sich beispielsweise, dass an Anlage B eine bessere Homogenisierung des Bunkermülls erreicht wird, da die Anzahl an Betriebsstunden mit geringer Variabilität des Abfallinputs (ausgedrückt durch die Standardabweichung des Anteils an biogenem Kohlenstoff über 4 h) deutlich höher ist (siehe Fig. 4).

Aus den Auswertungen zum Einfluss der zeitlichen Variabilität der Bunkermüllzusammensetzung (ausgedrückt durch die Standardabweichung des Anteils an biogenem Kohlenstoff über 4 h) auf den Anlagenbetrieb zeigt sich für Müllverbrennungsanlage A, dass bei höherer zeitlicher Variabilität der Abfallzusammensetzung (Standardabweichung von >5% des biogenen Kohlenstoffanteils: geringe Durchmischung/Homogenisierung des Bunkermülls) der mittlere Verbrauch an Heizöl zunimmt (von annähernd 0 auf 220 kg/h), die mittlere Dampfproduktion der Anlage abnimmt (von 106,2 t/h auf 102 t/h), der mittlere Abfalldurchsatz abnimmt (von 29 t/h auf 27,6 t/h) und gleichzeitig die mittlere Sauerstoffkonzentration im Abgas zunimmt (von 7,5 Vol.-% auf 8,05 Vol.-%), siehe Figs. 5 und 6. Alle beobachteten Auswirkungen führen zu finanziellen Einbußen für den Anlagenbetreiber.

Mithilfe des erfindungsgemäßen Verfahrens lassen sich diese Einbußen erstmalig quantifizieren und konkret mit der zeitlichen Variabilität der Zusammensetzung des Abfallinputs (Durchmischung/Homogenisierung des Bunkermülls) erklären, und dies praktisch in Echtzeit, was bis dato nicht möglich war.

Nutzung der Erfindung zur Ausweisung biogener und fossiler Energieträgeranteile sowie fossiler und biogener Kohlendioxidemissionen der Verbrennungsanlage:

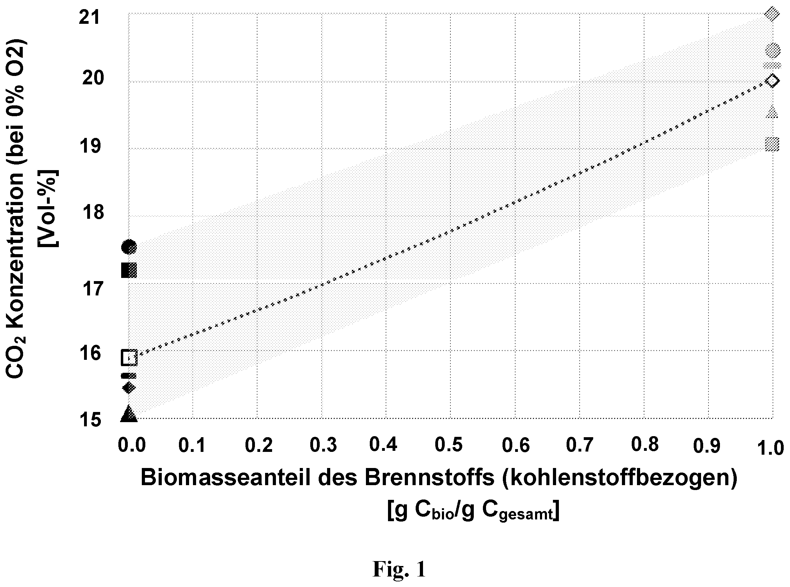

Das erfindungsgemäße Verfahren eignet sich nicht nur zur Betriebsoptimierung sondern kann auch näherungsweise zur Ausweisung biogener und fossiler Energieträgeranteile sowie fossiler und biogener Kohlendioxidemissionen der Verbrennungsanlage verwendet werden, beispielsweise unter Verwendung des in Fig. 1 dargestellten Zusammenhangs.

Die **Fig. 1** zeigt als punktierte Linie und, mögliche Abweichungen berücksichtigend, als grauen Streifen, die Abgaszusammensetzung (ausgedrückt in Form der $CO_{2Bezug}$ Konzentration bei stöchiometrischem Luftbedarf) über dem Biomasseanteil des verbrannten Abfalls (kohlenstoffbezogen in g $C_{bio}$/g $C_{gesamt}$); in Mischung mit verschiedenen fossilen Brennstoffen. Dazu sind angegeben:

Am linken Rand, von oben nach unten, einige fossile Brennstoffe in SCHWARZ:

Kreis: Polystyrol,
Quadrat: Polyvinylchlorid,
umrandetes Quadrat: typischer Mix an Kunststoffen in brennbaren Abfällen (Kunststoff-Mix),
Rechteck: Heizöl,
Raute: Polyamid,
Dreieck: Polyethylen und Polypropylen

und am rechten Rand, von oben nach unten, einige biogene Brennstoffe, in GRAU:

Raute: Zellulose,
Kreis: Holz,
Rechteck: Papier & Karton,
umrandete Raute: typischer Mix an biogenen Materialien in brennbaren Abfällen (Biogen-Mix),
Dreieck: Gartenabfälle,
Quadrat: Küchenabfälle

jeweils bei stöchiometrischem Luftbedarf.

Die Zahlen zur chemischen Zusammensetzung von Kunststoff-Mix bzw. Biogen-Mix entstammen Arbeiten, die im Zusammenhang mit der eingangs genannten EP 1 715 339: "Verfahren zur Ermittlung der Anteile biogener und fossiler Energieträger" oder danach durchgeführt wurden.

Dieser in **Fig. 1** dargestellte direkte Zusammenhang (Rückschluss) von $CO_{2Bezug}$ auf den Biomasseanteil wird insbesondere zur verständlicheren Kommunikation der erhaltenen Ergebnisse (zur Durchmischung und Steuerung des Bunkermülls) vorgeschlagen. Es ist für die Anwender der Erfindung, die Betreiber von Müllverbrennungsanlagen, leichter, sich die zeitliche Variabilität des Biomasseanteils im Müllinput vorzustellen, als die Tatsache, dass die Variabilität von $CO_{2Bezug}$ bereits eine direkte Aussage über die Variablität der Müllzusammensetzung liefert.

Die **Fig. 2** zeigt ein Beispiel zur zeitlich hochaufgelösten Analyse der Variabilität der Zusammensetzung des Abfallinputs (hier gemessen anhand der Standardabweichung des Biomasseanteils (kohlenstoffbezogen) inklusive Angabe jener Zeiträume (grau markierte Bereiche) in denen die Variabilität ein festgelegtes Maß (hier die Standardabweichung des Biomasseanteils über 40 min von 0,015 g $C_{bio}/C_{gesamt}$) übersteigt und somit der Bereich des optimalen Betriebes für die gegenständliche Anlage verlassen wird.

Es ist in diesen Fällen eine intensivere bzw. gezieltere Durchmischung des Bunkermülls gefordert, um einen optimalen Betrieb (max. Energieeffizienz, max. Mülldurchsatz und max. Dampfproduktion der Müllverbrennungsanlage) zu gewährleisten.

Legende:

strichlierte Linie: gemessene $O_2$ Konzentration im trockenen Abgas,
punktierte Linie: gemessene $CO_2$ Konzentration im trockenen Abgas,
schwarze Linie: berechnete $CO_2$ Konzentration im trockenen Abgas bei einem Bezugssauerstoffgehalt von 0 Vol.-%;
graue Linie mit Punktmarkierungen: errechneter Biomasseanteil des Brennstoffs (kohlenstoffbezogen) $C_{bio}/C_{gesamt}$,
graue Linie: errechnete Standardabweichung des Biomasseanteils (kohlenstoffbezogen und 10-fach überhöht dargestellt)

Die **Fig. 3** zeigt einerseits den aktuellen Stand der Technik zur zufälligen Mischung des Bunkermülls bzw. des aufgegebenen Brennstoffs ohne Kenntnis der räumlichen Verteilung der Abfallzusammensetzung im Bunker (linke Abbildung) und andererseits die durch die gegenständliche Erfindung ermöglichte gezielte und kontrollierte Mischung des Bunkermülls bzw. des aufgegebenen Brennstoffs bei Kenntnis der räumlichen Verteilung der Abfallzusammensetzung im Bunker (rechte Abbildung), wobei die gezielte und kontrollierte Mischung zu einer geringeren zeitlichen Variabilität der Brennstoffzusammensetzung führt.

Die **Fig. 4** zeigt in zwei Darstellungen eine Analyse der kurzzeitigen Variabilität der Abfallzusammensetzung für 2 Müllverbrennungsanlagen (Anlage A und Anlage B), ausgedrückt durch die Standardabweichung des Anteils an biogenem Kohlenstoff über 4 h [in %] und der jeweiligen Anzahl an Betriebsstunden, für die diese Variabilität in der Zusammensetzung beobachtet wurde.

Legende:
Eine Standardabweichung des Anteils an biogenen Kohlenstoff von <0,5% (Säule ganz rechts) repräsentiert eine sehr gute Durchmischung des Bunkermülls (geringe zeitliche Variabilität), während eine Standardabweichung von >5% (Säule ganz links) auf eine schlechte Durchmischung des Bunkermülls hindeutet (große zeitliche Variabilität der Abfallzusammensetzung).

Die **Fig. 5** zeigt in zwei Darstellungen für die Müllverbrennungsanlage A in Fig. 5a den mittleren Verbrauch am Zusatzbrennstoff Heizöl und in Fig. 5b die mittlere Dampfproduktion in Abhängigkeit der kurzzeitigen Variabilität (Schwankungen) der Abfallzusammensetzung (ausgedrückt durch die Standardabweichung des Anteils an biogenem Kohlenstoff über 4 h).

Legende:
Eine Standardabweichung des Anteils an biogenen Kohlenstoff von <0,5% (Säule ganz rechts) repräsentiert eine sehr gute Durchmischung des Bunkermülls (geringe zeitliche Variabilität), während eine Standardabweichung von >5% (Säule ganz links) auf eine schlechte Durchmischung des Bunkermülls hindeutet (große zeitliche Variabilität der Abfallzusammensetzung).

Die **Fig. 6** zeigt, ebenfalls in zwei Darstellungen für die Müllverbrennungsanlage A in Fig. 6a den mittleren Abfalldurchsatz und in Fig. 6b die mittlere Sauerstoffkonzentration im Abgas, wiederum in Abhängigkeit von der kurzzeitigen Variabilität (Schwankungen) der Abfallzusammensetzung (ausgedrückt durch die Standardabweichung des Anteils an biogenem Kohlenstoff über 4 h).

Legende:
Eine Standardabweichung des Anteils an biogenen Kohlenstoff von <0,5% (Säule ganz rechts) repräsentiert eine sehr gute Durchmischung des Bunkermülls (geringe zeitliche Variabilität), während eine Standardabweichung von >5% (Säule ganz links) auf eine schlechte Durchmischung des Bunkermülls hindeutet (große zeitliche Variabilität der Abfallzusammensetzung).

In einer Ausgestaltung der Erfindung wird für jede aufgegebene Schaufel (in den Brennraum eingebrachtes Quantum) deren Entnahmestelle im Bunker erfasst, was durch die Steuerung des Krans möglich ist. Bereits nach einer für jede Anlage charakteristischen, aber insgesamt kurzen Zeit wirkt sich die Abfallzusammensetzung der betrachteten Schaufel auf die Zusammensetzung der Abgase aus, sodass rasch eine ausreichend genaue Kenntnis über die Zusammensetzung des im Bunker an den jeweiligen Stellen gelagerten Mülls besteht. Durch die zeitliche Abfolge der Entnahmen und den geometrischen Zusammenhang wird dieses Wissen stets aktualisiert und berücksichtigt rasch auch Änderungen durch neu in den Bunker eingebrachten Müll. Es ist daher, anders als im Stand der Technik, nicht notwendig bei der Aufgabe des Mülls in den Brennraum von Vermutungen zur Bunkermüllzusammensetzung auszugehen, sondern es steht ein statistisch gesichertes und stets aktuelles Datenmaterial zur Verteilung der Abfälle im Bunker und deren Zusammensetzung zur Verfügung, das sowohl für die Brennstoffbeschickung als auch für die Durchmischung des Bunkermülls verwendet werden kann.

Durch diese Maßnahme gelingt es nicht nur, die Schwankungen geringer als im Stand der Technik zu halten, sondern auch, sie rascher, als es im Stand der Technik möglich ist, zu kompensieren.

Zusammenfassend ist zu sagen:
Die Erfindung betrifft ein Verfahren zur Analyse des Betriebs von Müllverbrennungsanlagen nach Anspruch 1.

Die Erfindung betrifft auch ein Verfahren zur Betriebsoptimierung von Müllverbrennungsanlagen nach Anspruch 2.

Eine (beliebige) Kombination dieser genannten Verfahren ist selbstverständlich möglich.

In einer Ausgestaltung dieser, gegebenenfalls kombinierten, Verfahren ist vorgesehen:

a) dass in einer Vorbereitungsphase die Zeit von der Einbringung eines Quantums an Müll in den Brennraum bis zur Erfassung im Abgas bestimmt wird,
b) im Betrieb der Ort der Entnahme jedes Quantums im Bunker bestimmt und gespeichert wird,
c) die Auswirkung jedes Quantums auf das Abgas, und damit das Verhältnis von biogenem zu fossilen Kohlenstoff an diesem Ort des Bunkers unter Berücksichtigung der im Schritt a) bestimmten Zeit bestimmt wird und
d) unter Berücksichtigung der in vorausgegangenen Schritten b) und c) der Ort im Bunker für die nächste Entnahme eines Quantums ausgewählt wird.
e) unter Berücksichtigung der in vorausgegangenen Schritten b) und c) die Orte im Bunker für die Durchmischung des Mülls (Aufnahme eines Quantums an einem Ort und Verstreuung dessen an einem anderen Ort im Bunker) ausgewählt wird.

In einer Weiterbildung ist vorgesehen, dass bei Einbringung neuen Mülls in den Bunker der Ort der Einbringung bestimmt und gespeichert wird und bis zur ersten Entnahme eines Quantums von diesem Ort das Verhältnis von biogenem zu fossilem Kohlenstoff an diesem Ort des Bunkers als unbekannt gespeichert wird.

In der Beschreibung und den Ansprüchen bedeutet "im Wesentlichen" eine Abweichung von bis zu 10 % des angegebenen Wertes, wenn es physikalisch möglich ist, sowohl nach unten als auch nach oben, ansonsten nur in die sinnvolle Richtung, bei Gradangaben (Winkel und Temperatur) sind damit ± 10° gemeint.

Alle Mengenangaben und Anteilsangaben, insbesondere solche zur Abgrenzung der Erfindung, soweit sie nicht die konkreten Beispiele betreffen, sind mit ± 10 % Toleranz zu verstehen, somit beispielsweise: 11 % bedeutet: von 9,9 % bis 12,1 %. Bei Bezeichnungen wie bei: "ein Lösungsmittel" ist das Wort "ein" nicht als Zahlwort, sondern als unbestimmter Artikel oder als Fürwort anzusehen, wenn nicht aus dem Zusammenhang etwas anderes hervorgeht.

Der Begriff: "Kombination" bzw. "Kombinationen" steht, sofern nichts anderes angegeben, für alle Arten von Kombinationen, ausgehend von zwei der betreffenden Bestandteile bis zu einer Vielzahl oder aller derartiger Bestandteile, der Begriff: "enthaltend" steht auch für "bestehend aus".

Die in den einzelnen Ausgestaltungen und Beispielen angegebenen Merkmale und Varianten können mit denen der anderen Beispiele und Ausgestaltungen frei kombiniert und ohne zwangsläufige Mitnahme der anderen Details der jeweiligen Ausgestaltung bzw. des jeweiligen Beispiels verwendet werden.

**Patentansprüche**

1. Verfahren zur Analyse des Betriebs von Müllverbrennungsanlagen, wobei im Abgas der Gehalt an $CO_2$ gemessen und nach Rückführung auf die Größe $CO_{2Bezug}$ das Verhältnis von biogenem zu fossilen Kohlenstoff im verbrannten Müll daraus bestimmt wird und die Variabilität von $CO_{2Bezug}$ bzw. des Verhältnisses von biogenem zu fossilen Kohlenstoff im verbrannten Müll nach Größe und Dauer bestimmt und aufgezeichnet werden.

2. Verfahren zur Betriebsoptimierung von Müllverbrennungsanlagen, wobei im Abgas der Gehalt an $CO_2$ gemessen und nach Rückführung auf die Größe $CO_{2Bezug}$ das Verhältnis-von biogenem zu fossilen Kohlenstoff im verbrannten Müll daraus bestimmt wird und die Variabilität von $CO_{2Bezug}$ bzw. des Verhältnisses von biogenem zu fossilen Kohlenstoff im verbrannten Müll nach Größe und Dauer bestimmt und zur Auswahl des der Verbrennung zuzuführenden Mülls herangezogen wird und / oder zur Durchmischung des im Annahmebunker lagernden Mülls verwendet wird.

3. Verfahren nach Anspruch 2, wobei

   a) in einer Vorbereitungsphase die Zeit von der Einbringung eines Quantums an Müll in den Brennraum bis zur Erfassung im Abgas bestimmt wird,
   b) im Betrieb der Ort der Entnahme jedes Quantums im Bunker bestimmt und gespeichert wird,
   c) $CO_{2Bezug}$ bzw. das Verhältnis von biogenem zu fossilen Kohlenstoff an diesem Ort des Bunkers durch die Auswirkung des Quantums auf das Abgas unter Berücksichtigung der im Schritt a) bestimmten Zeit bestimmt und gespeichert wird, und
   d) unter Berücksichtigung der in vorausgegangenen Schritten b) und c) der Ort im Bunker für die nächste Entnahme eines Quantums ausgewählt wird, und wobei
   e) unter Berücksichtigung der in vorausgegangenen Schritten b) und c) die Orte im Bunker für die Durchmischung des Mülls (Aufnahme eines Quantums an einem Ort und Verstreuung dessen an einem anderen Ort im Bunker) ausgewählt wird.

4. Verfahren nach Anspruch 3, wobei bei Einbringung neuen Mülls in den Bunker der Ort der Einbringung bestimmt und gespeichert wird, dass bis zur ersten Entnahme eines Quantums von diesem Ort $CO_{2Bezug}$ bzw. das Verhältnis von biogenem zu fossilem Kohlenstoff an diesem Ort des Bunkers als unbekannt gespeichert wird und dass nach der ersten Entnahme eines Quantums von diesem Ort das nach Schritt c) bestimmte Verhältnis gespeichert wird.

**Claims**

1. Method for analysing the operation of waste incineration plants, wherein the $CO_2$ content in the exhaust gas is measured and, after conversion to the $CO_{2reference}$ the ratio of biogenic to fossil carbon in the incinerated waste is determined from it, and the variability of $CO_{2reference}$ or the ratio of biogenic to fossil carbon in the incinerated waste is determined and recorded according to magnitude and duration.

2. Method for optimising the operation of waste incineration plants, whereby the $CO_2$ content in the exhaust gas is measured and, after conversion to the $CO_{2Reference}$ the ratio of biogenic to fossil carbon in the incinerated waste is determined from this, and the variability of $CO_{2reference}$ or the ratio of biogenic to fossil carbon in the incinerated waste is determined according to magnitude and duration and is used to select the waste to be fed into the incinerator and/or to mix the waste stored a the receiving bunker.

3. Method according to claim 2, wherein

   a) in a preparation phase, the time from the introduction of a quantity of waste into the combustion chamber to its detection in the exhaust gas is determined,
   b) during operation, the location of the removal of each quantity in the bunker is determined and stored,
   c) $CO_{2reference}$ or the ratio of biogenic to fossil carbon at this location in the bunker is determined by the effect of the quantity on the exhaust gas and stored, taking into account the time determined in step a), and
   d) considering steps b) and c) above, the location in the bunker for the next removal of a quantity is selected, and
   e) considering the locations in the bunker for mixing the waste by steps b) and c) (taking a quantum at one location and scattering it at another location in the bunker) are selected.

4. Method according to claim 3, wherein when new waste is introduced into the bunker, the location of introduction is

EP 3 987 229 B1

determined and stored, that until the first removal of a quantity from this location, $CO_{2reference}$ or the ratio of biogenic to fossil carbon at this location in the bunker is stored as unknown, and that after the first removal of a quantity from this location, the ratio determined according to step c) is stored.

**Revendications**

1. Procédé d'analyse du fonctionnement d'installations d'incinération de déchets, selon lequel on mesure la teneur en $CO_2$ dans les fumées rejetées et, après mise en référence avec la grandeur $CO_{2Bezug}$, on détermine, sur cette base, le rapport entre le carbone biogénique et le carbone fossile dans les déchets incinérés, et on détermine et on enregistre la variabilité du $CO_{2Bezug}$, respectivement du rapport du carbone biogénique au carbone fossile, dans les déchets incinérés, selon la grandeur et la durée.

2. Procédé d'optimisation du fonctionnement d'installations d'incinération de déchets, selon lequel on mesure la teneur en $CO_2$ dans les fumées rejetées et, après mise en référence avec la grandeur $CO_{2Be-zug}$, on détermine, sur cette base, le rapport entre le carbone biogénique et le carbone fossile dans les déchets incinérés, et on détermine la variabilité du $CO_{2Bezug}$, respectivement du rapport du carbone biogénique au carbone fossile, dans les déchets incinérés, selon la grandeur et la durée, et on l'utilise pour la sélection des déchets devant être acheminés à l'incinération et/ou bien on l'utilise pour mélanger les déchets stockés dans la fosse de réception.

3. Procédé selon la revendication 2, selon lequel

   a) dans une phase de préparation, on détermine le temps depuis l'introduction d'une quantité de déchets dans la chambre de combustion, jusqu'à la détection dans les fumées rejetées,
   b) lors du fonctionnement, on détermine et on enregistre le lieu de prélèvement de chaque quantité dans la fosse,
   c) on détermine et on enregistre le $CO_{2Bezug}$, respectivement le rapport du carbone biogénique au carbone fossile, dans ce lieu de la fosse, du fait de l'action de la quantité sur les fumées rejetées, en tenant compte du temps déterminé à l'étape a), et
   d) on sélectionne le lieu dans la fosse pour le prélèvement suivant d'une quantité, en tenant compte des étapes b) et c) précédentes, et selon lequel
   e) on sélectionne les lieux dans la fosse pour le mélange des déchets (prise en charge d'une quantité dans un lieu et répartition de celle-ci dans un autre lieu dans la fosse), en tenant compte des étapes b) et c) précédentes.

4. Procédé selon la revendication 3, selon lequel, lors de l'introduction de nouveaux déchets dans la fosse, on détermine le lieu de l'introduction et on l'enregistre, que jusqu'au premier prélèvement d'une quantité depuis ce lieu, le $CO_{2Bezug}$, respectivement le rapport du carbone biogénique au carbone fossile, dans ce lieu de la fosse est enregistré comme étant inconnu, et qu'après le premier prélèvement d'une quantité depuis ce lieu, on enregistre le rapport déterminé selon l'étape c).

9

Fig. 1

EP 3 987 229 B1

Fig. 2

11

**Variabilität des verbrannten Abfalls**

**Zufälliges Mischen:**

Verteilung der Abfallzusammensetzung im Bunker ist *unbekannt*

**Gezieltes Mischen:**

Verteilung der Abfallzusammensetzung im Bunker ist durch Anwendung der Methode *bekannt*

Zusammensetzung des Abfalls

**Müllbunker**

**Fig. 3**

EP 3 987 229 B1

Fig. 4a

EP 3 987 229 B1

# Müllverbrennungsanlage B

**Fig. 4b**

Fig. 5a

Fig. 5b

Fig. 6a

Fig. 6b

**EP 3 987 229 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1698827 A

- EP 1715339 A